# EUROPÄISCHE PATENTSCHRIFT

(11) **EP 2 376 177 B1**
(45) Veröffentlichungstag und Bekanntmachung des Hinweises auf die Patenterteilung: **16.01.2013**
(21) Anmeldenummer: 10760262.5
(22) Anmeldetag: 22.09.2010
(51) Int. Cl.: A61B 18/12, H01R 13/00, A61B 18/00

(54) **SCHLAUCHVERBINDER FÜR EIN HOCHFREQUENZ-CHIRURGIEGERÄT, HANDGRIFF FÜR EIN HF-CHIRURGIEGERÄT UND VERFAHREN ZUM VERBINDEN VON SCHLÄUCHEN FÜR EIN HF-CHIRURGIEGERÄT MIT EINEM DERARTIGEN SCHLAUCHVERBINDER**
TUBE CONNECTOR FOR A RADIO-FREQUENCY SURGICAL DEVICE, HANDLE FOR AN RF SURGICAL DEVICE, AND METHOD FOR CONNECTING TUBES FOR AN RF SURGICAL DEVICE TO SUCH A TUBE CONNECTOR
RACCORD DE FLEXIBLE POUR APPAREIL CHIRURGICAL HAUTE FRÉQUENCE, POIGNÉE POUR APPAREIL CHIRURGICAL HAUTE FRÉQUENCE, ET PROCÉDÉ DE RACCORDEMENT DE FLEXIBLES POUR APPAREIL CHIRURGICAL HAUTE FRÉQUENCE AU MOYEN D'UN TEL RACCORD DE FLEXIBLE

(30) Priorität: 24.09.2009 DE 102009042948; 06.11.2009 DE 102009052208
(43) Veröffentlichungstag der Anmeldung: 19.10.2011
(73) Patentinhaber: ERBE Elektromedizin GmbH, 72072 Tübingen (DE)
(72) Erfinder: KEGREIß, Horst, 72070 Tübingen-Hirschau (DE); SCHWAHN, Britta, 72074 Tübingen (DE); BESCH, Hansjörg Björn, 72810 Gomaringen (DE); BRODBECK, Achim, 72555 Metzingen-Neuhausen (DE)
(74) Vertreter: Bohnenberger, Johannes
(86) Internationale Anmeldenummer: PCT/EP2010/005807
(87) Internationale Veröffentlichungsnummer: WO 2011/035902

(56) Entgegenhaltungen:
- EP-A2- 0 164 993
- WO-A2-2007/002179
- US-A- 3 234 317
- US-A- 3 909 506
- US-A- 4 466 690

## Beschreibung

Die Erfindung betrifft einen Schlauchverbinder für ein HF-Chirurgiegerät, einen Handgriff für ein HF-Chirurgiegerät sowie ein Verfahren zum Verbinden von Schläuchen für ein HF-Chirurgiegerät mit einem derartigen Schlauchverbinder.

HF-Chirurgiegeräte, wie beispielsweise von der Anmelderin hergestellt und vertrieben, sind allgemein bekannt. Derartige Geräte umfassen üblicherweise einen HF-Generator, der einen hochfrequenten, therapeutisch wirksamen elektrischen Strom erzeugt. Die Applikation des HF-Stromes erfolgt bei den bekannten Geräten beispielsweise über eine Sonde, die in den Körper eines Patienten einführbar ist. Die Sonde umfasst einen geräteseitigen Schlauchabschnitt und einen sondenseitigen Schlauchabschnitt. Zwischen dem geräteseitigen Schlauchabschnitt und dem sondenseitigen Schlauchabschnitt ist typischerweise ein Handgriff angeordnet, der Bedienelemente für das HF-Chirurgiegerät umfasst.

Häufig kommen bei bekannten HF-Chirurgiegeräten bipolare Sonden zum Einsatz, die eine zusätzliche Gaszuführung umfassen, so dass eine HF-Stromapplikation unter Schutzgasatmosphäre erfolgen kann. Dazu weisen der sondenseitige Schlauchabschnitt und der geräteseitige Schlauchabschnitt jeweils eine Schlauchleitung auf, die im Bereich des Handgriffes pneumatisch verbunden sind. Ferner sind die elektrischen Leitungen, die zur Übertragung des HF-Stroms in die Sondenspitze erforderlich sind, im Bereich des Handgriffs miteinander elektrisch gekoppelt. Bei bipolaren Sonden sind dazu im Bereich des Handgriffes zwei separate elektrische Verbindungen erforderlich. Ein Verbinder für koaxiale kabel ist aus US 3 909 506 bekannt.

Die Verbindung der einzelnen elektrischen Leiter und der koaxial ineinander angeordneten Gasleitungen hat sich in der Praxis als schwierig und aufwändig erwiesen, insbesondere aufgrund der kleinen Dimensionen bei HF-chirurgischen Sondenschläuchen. Eine weitere Schwierigkeit ergibt sich aus den unterschiedlichen Dimensionen des sondenseitigen Schlauchabschnitts und des geräteseitigen Schlauchabschnitts. Die Verbindung der einzelnen Komponenten des geräteseitigen Schlauchabschnitts und des sondenseitigen Schlauchabschnitts nach bisherigen Konzepten weist ferner einen relativ komplizierten Aufbau mit einer relativ hohen Anzahl an einzelnen Bauteilen und einem entsprechend hohen Montageaufwand auf. Derartige Konzepte haben sich zudem als störungsanfällig erwiesen.

Es ist Aufgabe der Erfindung, einen Schlauchverbinder anzugeben, der einen einfachen Aufbau aufweist und mit dem der Montageaufwand bei der Herstellung von Verbindungen HF-chirurgischer Schlauchsysteme reduziert wird. Ferner liegt der Erfindung die Aufgabe zugrunde, einen Handgriff für ein HF-Chirurgiegerät und ein Verfahren zur Verbindung von Schläuchen für ein HF-Chirurgiegerät mit einem derartigen Schlauchverbinder anzugeben.

Erfindungsgemäß wird diese Aufgabe im Hinblick auf den Schlauchverbinder durch den Gegenstand des Patentanspruchs 1, im Hinblick auf den Handgriff durch den Gegenstand des Patentanspruchs 9 und im Hinblick auf das Verfahren durch den Gegenstand des Patentanspruchs 10 gelöst.

Die Erfindung beruht auf dem Gedanken, einen Schlauchverbinder für ein HF-Chirurgiegerät mit zwei Endhülsen anzugeben, die jeweils einen axialen Durchgangskanal aufweisen und durch einen Steg miteinander verbunden sind. Der Steg erstreckt sich aus einer Wandungsebene der ersten Endhülse axial in Richtung der zweiten Endhülse. Zumindest der Steg umfasst wenigstens abschnittsweise ein elektrisch leitendes Material. Die Endhülsen sind zur Fixierung eines im jeweiligen Durchgangskanal anordnbaren Schlauches jeweils bereichsweise komprimierbar oder crimpbar.

Der erfindungsgemäße Schlauchverbinder ermöglicht eine einfache und sichere elektrische und gleichzeitig pneumatische Verbindung von Schlauchleitungen eines Hochfrequenzchirurgiegerätes. Dabei weist der Schlauchverbinder einen relativ einfachen Aufbau auf, so dass die Montage des Hochfrequenzchirurgiegeräts vereinfacht ist. Insbesondere wird die gasdichte pneumatische Kontaktierung jeweils durch Kompression bzw. Crimpung der Endhülsen erreicht. Die elektrische Kontaktierung kann ebenfalls durch die Crimpung hergestellt werden, wobei die Erfindung nicht darauf eingeschränkt ist. Die elektrische und pneumatische Verbindung kann also gleichzeitig mit wenigen Montageschritten, nämlich der Crimpung bzw. Klemmung, hergestellt und der Zeitaufwand für die Montage des Hochfrequenzchirurgiegeräts reduziert werden. Insbesondere durch die einfache Kontaktierung der elektrischen Leiter mit dem Schlauchverbinder wird eine Schlauchverbindung auf relativ kleinem Raum ermöglicht. Der Platzbedarf für die Schlauchverbindung, beispielsweise in einem Handgriff, wird somit reduziert. Überdies sind diese Vorteile mit nur einem einzigen Bauteil, nämlich dem erfindungsgemäßen Schlauchverbinder, erreichbar, wogegen bisherige Verbindungskonzepte zur Verbindung der elektrischen und pneumatischen Schlauchbestandteile auf eine Mehrzahl von unterschiedlichen Verbindungselementen zurückgegriffen haben.

Durch das elektrisch leitende Material des Schlauchverbinders wird eine sichere und stabile elektrische Verbindung zwischen dem Geräteschlauch und dem Sondenschlauch gewährleistet. Zusätzlich ermöglicht das elektrisch leitende Material eine Reduktion des Montageaufwandes, da die elektrische Verbindung zwischen dem Geräteschlauch und dem Sondenschlauch nicht, wie bisher üblich, direkt, sondern vielmehr mittelbar durch den Schlauchverbinder herstellbar ist.

Bei einer bevorzugten Ausführungsform des erfindungsgemäßen Schlauchverbinders umfasst die erste Endhülse einen sondenseitigen Abschnitt und einen geräteseitigen Abschnitt, wobei der sondenseitige Abschnitt einen kleineren Querschnittsdurchmesser als der geräteseitige Abschnitt aufweist. Diese Ausführungsform ermöglicht besonders vorteilhaft die Überführung eines Geräteschlauchs mit einem relativ größeren Querschnittsdurchmesser in einen Sondenschlauch mit einem relativ kleineren Durchmesser. Mit anderen Worten kann mit dem erfindungsgemäßen Schlauchverbinder ein Sondenschlauch mit einem Geräteschlauch des HF-Chirurgiegeräts verbunden werden, auch wenn der Sondenschlauch einen wesentlich kleineren Querschnittsdurchmesser als der Geräteschlauch aufweist. Der Schlauchverbinder gewährleistet dabei gleichzeitig eine druckfeste, d.h. leckagearme bzw. leckagefreie, pneumatische und eine sichere und stabile elektrische Verbindung zwischen dem Sondenschlauch und dem Geräteschlauch.

Ferner kann die erste Endhülse einen größeren Querschnittsdurchmesser als die zweite Endhülse aufweisen. Insbesondere der geräteseitige Abschnitt der ersten Endhülse weist einen größeren Querschnittsdurchmesser als die zweite Endhülse auf. Auf diese Weise wird ermöglicht, dass der Schlauchverbinder eine einfache Verbindung zwischen einem sondenseitigen Schlauch und einem geräteseitigen Schlauch, der als Koaxialleitung ausgebildet ist, erreicht. Als Koaxialleitung wird in diesem Zusammenhang eine Anordnung zweier schlauchartiger Leitungen verstanden, wobei eine innere, schlauchartige Leitung koaxial innerhalb einer äußeren schlauchartigen Leitung geführt ist. Dabei ist zwischen der inneren Leitung und der äußeren Leitung ein Freiraum gebildet. In dieser Konstellation ermöglicht der unterschiedliche Querschnittsdurchmesser der zweiten Endhülse und der ersten Endhülse, insbesondere in Verbindung mit dem die Endhülsen verbindenden Steg, die separate Kopplung sowohl des Lumens der äußeren Leitung des Geräteschlauchs mit dem Sondenschlauch, als auch des Lumens der inneren Leitung des Geräteschlauchs mit dem Sondenschlauch.

Vorzugsweise umfassen die zweite Endhülse und/oder der Steg eine Bohrung, die sich zumindest teilweise in radialer Richtung erstreckt. Die Bohrung ermöglicht auf besonders einfache Weise die Kontaktierung eines elektrischen Leiters mit dem elektrisch leitenden Schlauchverbinder. Beispielsweise kann die Bohrung derart ausgerichtet sein, dass ein elektrischer Leiter, wenn er von außen durch die Bohrung eingeführt wird, in den zweiten Durchgangskanal hineinragt. Durch das Einführen eines inneren Koaxialschlauchs in den zweiten Durchgangskanal wird der elektrische Leiter dann umgebogen und zwischen dem inneren Koaxialschlauch und dem Schlauchverbinder verklemmt. Alternative Verbindungsmöglichkeiten, beispielsweise Löten oder Kleben sind möglich.

Eine weitere vorteilhafte Ausgestaltung der Erfindung sieht vor, dass die Bohrung im Steg, der sich zwischen der ersten und der zweiten Endhülse erstreckt, mit einer axialen Begrenzungsfläche der zweiten Endhülse fluchtet, die der ersten Endhülse zugewandt ist. Eine derartige Anordnung der Bohrung ist sowohl fertigungstechnisch bei der Herstellung des Schlauchverbinders als auch montagetechnisch bei der Konnektion der Schläuche des HF-Chirurgiegeräts vorteilhaft. Insbesondere wird durch diese besondere Anordnung der Bohrung eine einfache elektrische Verbindung zwischen einem ersten elektrischen Leiter des Geräteschlauchs und dem Schlauchverbinder bzw. folglich dem Sondenschlauch ermöglicht.

Vorzugsweise umfasst die erste Endhülse eine Schulter, die zwischen dem sondenseitigen Abschnitt und dem geräteseitigen Abschnitt angeordnet ist und einen größeren Querschnittsdurchmesser als der geräteseitige Abschnitt aufweist. Durch den größeren Querschnittsdurchmesser im Vergleich zu dem geräteseitigen Abschnitt der ersten Endhülse kann die Schulter ein Verbindungselement zur Verbindung des Schlauchverbinders mit umgebenden Teilen, beispielsweise einem Handgriff für ein HF-Chirurgiegerät, bilden. Die besondere Anordnung der Schulter zwischen dem geräteseitigen Abschnitt und dem sondenseitigen Abschnitt ist fertigungstechnisch vorteilhaft, insbesondere wenn der sondenseitige Abschnitt einen kleineren Querschnittsdurchmesser als der geräteseitige Abschnitt aufweist.

Gemäß einer weiteren bevorzugten Ausführungsform des erfindungsgemäßen Schlauchverbinders kann eine äußere Crimphülse vorgesehen sein, die den geräteseitigen Abschnitt der ersten Endhülse ringförmig umgreift und zur Fixierung eines geräteseitigen Schlauches zumindest bereichsweise radial komprimierbar ist. Die äußere Crimphülse ermöglicht eine einfache Fixierung des Geräteschlauchs an dem Schlauchverbinder, insbesondere dem geräteseitigen Abschnitt der ersten Endhülse durch eine radiale Verformung. Diese als Crimpen bzw. Klemmen bezeichnete Verformung bildet somit eine kraft- und formschlüssige Verbindung zwischen der äußeren Crimphülse, dem Geräteschlauch und dem geräteseitigen Abschnitt der ersten Endhülse.

Gemäß einem nebengeordneten Aspekt beruht die Erfindung auf dem Gedanken, ein Schlauchsystem für ein HF-Chirurgiegerät mit einem Schlauchverbinder gemäß Anspruch 1 anzugeben.

Ein weiterer nebengeordneter Aspekt der Erfindung betrifft ein Verfahren zum Verbinden von Schläuchen für ein Hochfrequenzchirurgiegerät mit einem Schlauchverbinder gemäß Anspruch 1, das die folgenden Schritte umfasst:
- Ein Sondenschlauch wird in einem ersten Durchgangskanal der ersten Endhülse und ein innerer Geräteschlauch in einem zweiten Durchgangskanal der zweiten Endhülse angeordnet;
- ein erster elektrischer Leiter wird zwischen dem Steg und dem inneren Geräteschlauch angeordnet; und
- in die erste Endhülse werden wenigstens eine, insbesondere zwei, erste gasdichte Verbindungen und in die zweite Endhülse wenigstens eine zweite gasdichte Verbindung eingebracht.

Bezüglich der Wirkungen und Vorteile des erfindungsgemäßen Verfahrens wird auf die Ausführungen zum eingangs beschriebenen Schlauchverbinder verwiesen.

Bei einer bevorzugten Ausführungsform umfassen die erste gasdichte Verbindung eine erste Crimpung und/oder die zweite gasdichte Verbindung eine zweite Crimpung. Die Nutzung eines Crimpvorgangs zur Herstellung der gasdichten Verbindungen ist insbesondere bei Herstellung der Schlauchverbindung unter Reinraumbedingungen vorteilhaft. Überdies ermöglicht die Crimpung gleichzeitig eine Klemmung der elektrischen Verbindungen, so dass ein zusätzlicher Arbeitsschritt eingespart wird.

Der erste elektrische Leiter kann über eine axiale Begrenzungsfläche der zweiten Endhülse, die der ersten Endhülse zugewandt ist, oder eine Bohrung, die sich zumindest teilweise in radialer Richtung durch die zweite Endhülse oder den Steg erstreckt, in den zweiten Durchgangskanal eingeführt werden. In jedem Falle ist es zweckmäßig, wenn der zweite elektrische Leiter innerhalb der zweiten Endhülse, vorzugsweise im zweiten Durchgangskanal, angeordnet wird und der erste elektrische Leiter innerhalb der zweiten Endhülse, beispielsweise zwischen der zweiten Endhülse und einem inneren Geräteschlauch fixiert wird. Die Fixierung kann beispielsweise durch Klemmung erfolgen. Es ist jedoch nicht ausgeschlossen, dass der erste elektrische Leiter auch durch die Crimpung der zweiten Endhülse oder eine stoffschlüssige Verbindung, beispielsweise eine Löt- oder Klebeverbindung, fixiert wird.

Bei einer weiteren bevorzugten Ausführungsform des erfindungsgemäßen Verfahrens wird ein äußerer Geräteschlauch über einen geräteseitigen Abschnitt der ersten Endhülse geführt und mit einer Crimphülse umschlossen, in die zur Fixierung des äußeren Geräteschlauchs eine dritte Crimpung eingebracht wird.

Eine weitere bevorzugte Ausgestaltung des Verfahrens sieht vor, dass ein zweiter elektrischer Leiter durch eine Öffnung des inneren Geräteschlauchs geführt und mit einem Düsenrohr elektrisch verbunden wird, das sich koaxial von einem Sondenschlauch in den inneren Geräteschlauch erstreckt.

Die elektrischen Verbindungen zwischen dem Geräteschlauch und dem Sondenschlauch werden vorzugsweise durch Klemmung fixiert. Die elektrischen Leiter werden also zwischen dem Schlauchverbinder und dem Geräteschlauch, insbesondere zwischen dem inneren Geräteschlauch und der zweiten Endhülse, geklemmt. Durch die Crimpung der zweiten Endhülse wird die Klemmverbindung fixiert.

Die Erfindung wird im Folgenden unter Bezugnahme auf die beigefügten, schematischen Zeichnungen näher erläutert. Darin zeigen
- Fig. 1: einen Längsschnitt durch einen erfindungsgemäßen Schlauchverbinder gemäß einem bevorzugten Ausführungsbeispiel; und
- Fig. 2 und 3: unterschiedliche Verfahrensschritte zur Verbindung von Schläuchen eines HF-Chirurgiegerätes mit dem Schlauchverbinder gemäß Fig. 1.

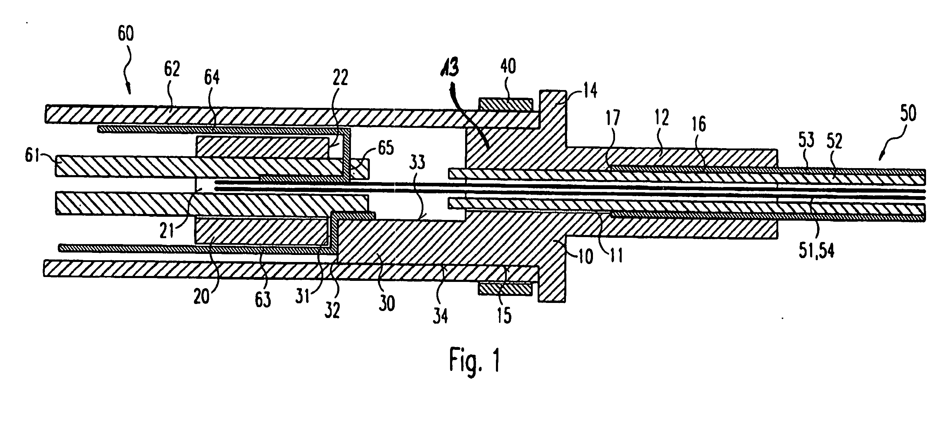

Der Schlauchverbinder gemäß dem Ausführungsbeispiel nach Figur 1 umfasst eine erste Endhülse 10 und eine zweite Endhülse 20, wobei die Endhülsen 10, 20 durch einen Steg 30 miteinander verbunden sind. Der Schlauchverbinder, insbesondere die Endhülsen 10, 20 und der Steg 30, ist einteilig ausgebildet. Die Endhülsen 10, 20 weisen jeweils eine rotationssymmetrische Form auf und sind zueinander koaxial ausgerichtet. Insbesondere umfassen die Endhülsen 10, 20 jeweils einen Durchgangskanal 11, 21, wobei die Durchgangskanäle 11, 21 zueinander fluchtend bzw. koaxial angeordnet sind. In Längsrichtung des Schlauchverbinders sind die Endhülsen 10, 20 voneinander beabstandet angeordnet, wobei der Abstand der Endhülsen 10, 20 durch die Länge des Stegs 30 bestimmt ist.

Die erste Endhülse 10 umfasst einen ersten Durchgangskanal 11, in dem im Gebrauch ein Sondenschlauch 50 angeordnet bzw. anordenbar ist. Dabei entspricht der Querschnittsdurchmesser des ersten Durchgangskanals 11 im Wesentlichen dem Querschnittsdurchmesser des Sondenschlauchs 50. Im Bereich eines axialen äußeren Endes des Schlauchverbinders weist die erste Endhülse 10 eine Axialbohrung 16 auf, die sich über einen Teil der Länge des ersten Durchgangskanals 11 erstreckt. Die Axialbohrung 16 weist einen größeren Querschnittsdurchmesser auf als der erste Durchgangskanal 11 und bildet im Wesentlichen einen aufgeweiteten Abschnitt des ersten Durchgangskanals 11. Die axiale Begrenzung der Axialbohrung 16 bildet einen Anschlag 17, der den Übergang zwischen dem relativ kleineren Querschnittsdurchmesser des ersten Durchgangskanals 11 und dem relativ größeren Querschnittsdurchmesser der Axialbohrung 16 bildet. Im Gebrauch, also wenn der Schlauchverbinder mit einem Sondenschlauch 50 gekoppelt ist, nimmt die Axialbohrung 16 einen ersten Sondenleiter 53 des Sondenschlauchs 50 auf.

Der erste Sondenleiter 53 bildet die radiale Umfangsbegrenzung des Sondenschlauchs 50. Innerhalb des ersten Sondenleiters 53 ist ein äußerer Sondenschlauch 52 angeordnet, der als Gasrückführung dient. Für die Gaszuführung zur Sonde umfasst der Sondenschlauch 50 ferner ein Düsenrohr 51, das koaxial innerhalb des Sondenschlauchs 50 angeordnet ist und einen wesentlich kleineren Querschnittsdurchmesser als der äußere Sondenschlauch 52 aufweist. Das Düsenrohr 51 bildet im Wesentlichen einen inneren Sondenschlauch. Auf diese Weise werden mit dem Sondenschlauch 50 zwei koaxial ineinander angeordnete Gasleitungen bereitgestellt. Dabei dient das Düsenrohr 51 ferner als zweiter Sondenleiter 54. Die Sondenleiter 53, 54 bilden die elektrische Verbindung zu bipolaren Elektroden an der Sondenspitze. Die Sondenleiter 53, 54 weisen also ein elektrisch leitendes Material auf. Über das Düsenrohr 51 wird dabei die Zuführung eines Kühlmediums in den Bereich der Sondenspitze bzw. in den Behandlungsbereich ermöglicht. Über die durch den äußeren Sondenschlauch 52 gebildete Gasleitung ist eine Rückführung des Kühlmediums zum HF-Chirurgiegerät möglich.

In diesem Zusammenhang wird darauf hingewiesen, dass im Falle der Verwendung eines Schutzgases anstelle eines Kühlmediums eine Gasrückführung nicht zwingend erforderlich ist. Es ist also möglich, dass bei Verwendung eines Schutzgases lediglich eine Gaszuführung, beispielsweise über das Düsenrohr 51, vorgesehen ist. Das Schutzgas entweicht an der Sondenspitze und wird am Behandlungsort freigesetzt. Hingegen ist es zweckmäßig, bei Verwendung eines Kühlmediums, insbesondere eines Kühlgases, die Gasrückführung vorzusehen, um das Kühlmedium in einem Kühlkreislauf durch die Sondenspitze strömen zu lassen.

Neben dem sondenseitigen Abschnitt 12, der die Axialbohrung 16 umfasst, weist die erste Endhülse 10 einen geräteseitigen Abschnitt 13 auf, der durch eine Schulter 14 von dem sondenseitigen Abschnitt 12 getrennt ist. Der geräteseitige Abschnitt 13 umfasst einen größeren Querschnittsdurchmesser als der sondenseitige Abschnitt 12. Die Schulter 14, die zwischen dem geräteseitigen Abschnitt 13 und dem sondenseitigen Abschnitt 12 angeordnet ist, weist wiederum einen größeren Querschnittsdurchmesser als der geräteseitige Abschnitt 13 auf. Die Schulter 14 bildet also eine über den Umfang des Schlauchverbinders umlaufende Rippe, die die Verbindung des Schlauchverbinders mit weiteren äußeren Bauteilen, beispielsweise einem Handgriff eines HF-Chirurgiegerätes ermöglicht.

Von dem geräteseitigen Abschnitt 13 erstreckt sich der Steg 30 in Richtung der zweiten Endhülse 20. Der Steg 30 weist dabei eine Außenfläche 34 auf, die mit einer äu-βeren Umfangsfläche 15 des geräteseitigen Abschnitts 13 fluchtet. In Umfangsrichtung erstreckt sich der Steg 30 teilweise über den Umfang des geräteseitigen Abschnitts 13. Der Steg 30 ist also im Wesentlichen aus einem Kreissegment des geräteseitigen Abschnitts 13 gebildet, das in Richtung der zweiten Endhülse 20 verlängert ist. Ferner weist der Steg 30 eine Innenfläche 33 auf, die mit einem zweiten Durchgangskanal 21 der zweiten Endhülse 20 fluchtet. Dabei kann die Innenfläche 33 entsprechend die in Umfangsrichtung ausgebildete Krümmung des zweiten Durchgangskanals 21 nachbilden. Alternativ ist es auch möglich, dass die Innenfläche 33 flächig bzw. flach ausgebildet ist und im Wesentlichen tangential zur Krümmung des zweiten Durchgangskanals 21 ausgerichtet ist.

An einem der zweiten Endhülse 20 zugewandten Längsende des Stegs 30 ist ein Absatz 32 gebildet, der sich aus den unterschiedlichen Querschnittsdurchmessern der Endhülsen 10, 20 ergibt. Insbesondere weist die zweite Endhülse 20 einen kleineren Querschnittsdurchmesser als die erste Endhülse 10 auf. Dem Steg 30 ist die radiale Bohrung 31 zugeordnet, die einerseits mit dem Absatz 32 und andererseits mit einer axialen Begrenzungsfläche 22 der zweiten Endhülse 20 fluchtet. Dabei bezieht sich die fluchtende Anordnung der Bohrung 31 auf die den Bohrungsdurchmesser definierende Innenfläche der Bohrung und die entsprechend tangential dazu angeordnete axiale Begrenzungsfläche 22 bzw. dem tangential dazu ausgerichteten Absatz 32.

Alternativ kann die Bohrung 31 einen schrägen Anstellwinkel aufweisen, so dass die Bohrungsachse der Bohrung 31 zur Längsachse der Durchgangskanäle 11, 21 einen Winkel bildet, der ungleich 90° ist. Vorzugsweise ist die Bohrung 31 in diesem Fall derart ausgerichtet, dass sich die Bohrungsachse im Wesentlichen in Richtung der ersten Endhülse 10 erstreckt. Auf diese Weise wird erreicht, dass ein elektrischer Leiter, der durch die Bohrung 31 in den Bereich des zweiten Durchgangskanals bzw. in den Bereich zwischen dem ersten Durchgangskanal 11 und dem zweiten Durchgangskanal 21 hineinerstreckt, durch den inneren Geräteschlauch 61 einfach abgeknickt und geklemmt werden kann.

Die Bohrung 31 dient also der einfachen elektrischen Verbindung zwischen einem ersten elektrischen Leiter 63 und dem Schlauchverbinder. In alternativen Ausführungsformen ist eine Bohrung 31 nicht vorgesehen. Beispielsweise kann stattdessen der erste elektrische Leiter 63 über die axiale Begrenzungsfläche 22 in den zweiten Durchgangskanal 21 der zweiten Endhülse 20 eingeführt werden. Dabei kann der erste elektrische Leiter 63 im zweiten Durchgangskanal mit dem Schlauchverbinder verklemmt, verlötet, verklebt oder anderweitig elektrisch verbunden werden.

Es wird darauf hingewiesen, dass der Übergang zwischen den einzelnen Abschnitten des Schlauchverbinders, insbesondere der Übergang vom Steg 30 zur zweiten Endhülse 20, im Allgemeinen unterschiedlich gestaltbar ist. Beispielsweise kann der Steg 30 zur Endhülse 20 hin auslaufen, so dass der Absatz 32 im Wesentlichen schräg verläuft bzw. eine Rundung oder eine Fase bildet. Der Absatz 32 kann kegelsegmentförmig ausgebildet sein. Es ist ferner möglich, dass der Steg 30, insbesondere wenn dieser als Fortsetzung eines Kreissegments des geräteseitigen Abschnitts 13 gebildet ist, zur jeweiligen Endhülse 10, 20 einen Übergang bildet, bei dem sich das in den Querschnitt des Stegs 30 abbildende Kreissegment vergrößert. Mit anderen Worten kann der Steg 30 auch kanuförmig ausgebildet sein.

Die zweite Endhülse 20 ist im Wesentlichen zylinderförmig bzw. rohrartig ausgebildet. Die zweite Endhülse 20 bildet also im Wesentlichen ein rohrartiges Bauteil, das den zweiten Durchgangskanal 21 umfasst. Im Gebrauch ist der Geräteschlauch 60, insbesondere ein innerer Geräteschlauch 61, in der zweiten Durchgangsbohrung 21, also in der zweiten Endhülse 20, angeordnet bzw. anordnbar. Ferner ist die zweite Endhülse 20 im Gebrauch innerhalb eines äußeren Geräteschlauchs 62 des Geräteschlauchs 60 angeordnet bzw. anordnbar.

Die Verbindung eines Geräteschlauchs 60 und eines Sondenschlauchs 50 mit dem Schlauchverbinder wird folgendermaßen bewerkstelligt:

Der Geräteschlauch 60 umfasst einen inneren Geräteschlauch 61 und einen äußeren Geräteschlauch 62, wobei der innere Geräteschlauch 61 koaxial innerhalb des äußeren Geräteschlauchs 62 angeordnet ist. Der zwischen dem inneren Geräteschlauch 61 und dem äußeren Geräteschlauch 62 gebildete Freiraum dient dabei als Gasrückführung, wogegen das Lumen des inneren Geräteschlauchs 61 eine Gaszuführung bildet. Ferner sind im Zwischenraum zwischen dem inneren und dem äußeren Geräteschlauch 61, 62 zwei elektrische Leiter 63, 64 angeordnet. Die elektrischen Leiter 63, 64 sind mit dem HF-Chirurgiegerät zur Übertragung einer elektrischen Energie in die HF-Sonde elektrisch gekoppelt. Die weitere Verbindung der elektrischen Leiter 63, 64 mit den entsprechenden Sondenleitern 53, 54 erfolgt über den Schlauchverbinder, der ein elektrisch leitendes Material umfasst. Dabei wird der erste elektrische Leiter 63 von außen durch die Bohrung 31 geführt, so dass ein Endabschnitt des ersten elektrischen Leiters 63 vor dem zweiten Durchgangskanal 21 der zweiten Endhülse 20 zu liegen kommt. Alternativ wird der erste elektrische Leiter 63 direkt in den zweiten Durchgangskanal 21 eingeführt bzw. eingelegt, wobei der Zugang zum zweiten Durchgangskanal 21 sowohl über die der ersten Endhülse 10 zugewandten Begrenzungsfläche 22, als auch über eine axiale Endfläche 23 der zweiten Endhülse 20 erfolgen kann. Daraufhin wird der innere Geräteschlauch 61 durch den zweiten Durchgangskanal 21 geführt, wobei der innere Geräteschlauch 61 nach Durchtritt durch die zweite Endhülse 20 den Endabschnitt des ersten elektrischen Leiters 63 umknickt, so dass dieser zwischen dem inneren Geräteschlauch 61 und der Innenfläche 33 des Stegs 30 geklemmt wird. Im Folgenden wird der zweite elektrische Leiter 64 durch eine radiale Öffnung 65 geführt, die in einem Endabschnitt des inneren Geräteschlauchs 61 angeordnet ist. Dabei wird der zweite elektrische Leiter 64 von außen durch die radiale Öffnung 65 geführt, bis ein Endabschnitt des zweiten elektrischen Leiters 64 innerhalb des Lumens des inneren Geräteschlauchs 61 zu liegen kommt. Analog zur alternativen Verbindung des ersten elektrischen Leiters 63 kann der zweite elektrische Leiter 64 auch über eine Stirnfläche des inneren Geräteschlauchs 61 in das Lumen des inneren Geräteschlauchs 61 eingeführt werden.

In einem weiteren, nachgeordneten Schritt wird dann der äußere Geräteschlauch 62 über die zweite Endhülse 20, dem Steg 30 und abschließend den geräteseitigen Abschnitt 13 der ersten Endhülse 10 geführt. Die Schulter 14 der ersten Endhülse 10 bildet dabei einen Anschlag für den äußeren Geräteschlauch 62. Wie in Fig. 1 ferner dargestellt, ist zur Verbindung bzw. Fixierung des Geräteschlauchs 60 mit dem Schlauchverbinder ferner eine Crimphülse 40 vorgesehen, die ringförmig ausgebildet ist und im Bereich des geräteseitigen Abschnitts 13 angeordnet ist. Die Crimphülse 40 umgreift dabei den äußeren Geräteschlauch 62. Das bedeutet, dass die Crimphülse 40 einen größeren Querschnittsdurchmesser aufweist als der geräteseitige Abschnitt 13 der ersten Endhülse 10. Ferner weist die Crimphülse 40 einen kleineren Querschnittsdurchmesser auf als die Schulter 14, so dass die Schulter 14 auch für die Crimphülse 40 einen Anschlag bildet. Gemäß dem in den Figuren dargestellten Ausführungsbeispiel besteht zwischen der Crimphülse 40 und der ersten Endhülse 10 keine Verbindung. Es ist aber auch möglich, dass die Crimphülse 40 mit der Schulter 14 der ersten Endhülse 10 fest oder lösbar gekoppelt ist. Beispielsweise kann die Crimphülse 40 einteilig mit der Schulter 14 ausgebildet sein, so dass die Crimphülse 40 im Wesentlichen eine ringförmige Rippe bildet, die in axialer Richtung von der Schulter 14 ausgeht. Die Crimphülse 14 begrenzt in diesem Fall eine ringförmige Nut, in die der äußere Geräteschlauch 62 eingeführt werden kann.

Vor oder nach der Verbindung des äußeren Geräteschlauchs 62 mit der ersten Endhülse 10 wird der Sondenschlauch 50 mit dem Schlauchverbinder gekoppelt. Der Sondenschlauch 50 umfasst den äußeren Sondenschlauch 52, der einen auf dem Außenumfang angeordneten ersten Sondenleiter 53 aufweist, und das Düsenrohr 51, das gleichzeitig als zweiter Sondenleiter 54 dient. Wie zuvor beschrieben, wird der äußere Sondenschlauch 52 in den ersten Durchgangskanal 11 der ersten Endhülse 10 eingeführt, wobei der erste Sondenleiter 53 in der Axialbohrung 16 angeordnet wird. Der äußere Sondenschlauch 52 bzw. allgemein der Sondenschlauch 50 wird vorzugsweise derart in dem ersten Durchgangskanal 11 angeordnet, dass ein Endabschnitt des äußeren Sondenschlauchs 52 über den geräteseitigen Abschnitt 13 der ersten Endhülse 10 hervorragt. Innerhalb des äußeren Sondenschlauchs 52 ist das Düsenrohr 51 angeordnet, das sich über den äußeren Sondenschlauch 52 hinaus erstreckt. In Richtung des Geräteschlauchs 60 ragt also das Düsenrohr 51 über den äußeren Sondenschlauch 52 hervor. Das ermöglicht, dass das Düsenrohr 51 sich über den Abstand zwischen den Endhülsen 10, 20 des Schlauchverbinders in den inneren Geräteschlauch 61 erstreckt. Beim Einführen des Düsenrohrs 51 in den inneren Geräteschlauch 61 wird dabei der Endabschnitt des zweiten elektrischen Leiters 64 des Geräteschlauchs 60 abgeknickt und zwischen dem Düsenrohr 51 und dem inneren Geräteschlauch 61 eingeklemmt. Das Düsenrohr 51 weist ein elektrisch leitendes Material auf und stellt in der in Fig. 1 dargestellten Anordnung eine elektrische Verbindung zwischen der Sondenspitze und dem zweiten elektrischen Leiter 64 dar.

In diesem Zusammenhang wird darauf hingewiesen, dass der Abstand zwischen dem Düsenrohr 51 und dem inneren Geräteschlauch 61 in radialer Richtung aus zeichnerischen Gründen dargestellt ist. Tatsächlich ist vorgesehen, dass das Düsenrohr 51 mit dem inneren Geräteschlauch 61 im Wesentlichen fluiddicht bzw. druckdicht verbunden wird.

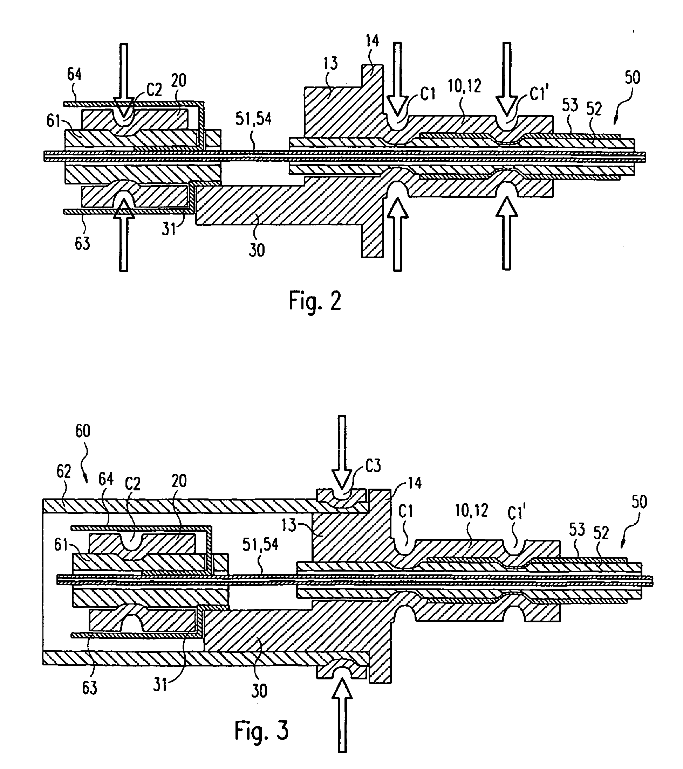

Um die Verbindung zwischen dem Geräteschlauch 60 und dem Sondenschlauch 50 mittels des Schlauchverbinders zu fixieren, werden an ausgewählten Punkten des Schlauchverbinders Crimpungen, also kraft- und formschlüssige Verbindungen, gesetzt. Bevorzugte Verbindungen sind in den Figuren 2 und 3 dargestellt. Insbesondere zeigt Figur 2 zwei erste Crimpungen C1, C1', die im Bereich des sondenseitigen Abschnitts 12 der ersten Endhülse 10 eingebracht werden. Eine andere Anzahl an Crimpungen, insbesondere eine einzige Crimpung oder mehr als zwei Crimpungen sind möglich. Bei dem Ausführungsbeispiel gemäß Figur 2 ist vorgesehen, dass eine der ersten Crimpungen C1 in einem an die Schulter 14 angrenzenden Bereich des sondenseitigen Abschnitts 12 eingebracht wird. Eine weitere erste Crimpung C1 wird im Bereich der Axialbohrung 16 des sondenseitigen Abschnitts 12 eingebracht. Insbesondere mit der weiteren ersten Crimpung C1' wird eine feste Verbindung zwischen dem ersten Sondenleiter 53 und dem Schlauchverbinder hergestellt. Im weiteren Verlauf wird eine zweite Crimpung C2 in die zweite Endhülse 20 eingebracht, um den inneren Geräteschlauch 61 in der zweiten Endhülse 20 zu fixieren. Der äußere Geräteschlauch 62 wird durch eine dritte Crimpung C3 mit der ersten Endhülse 10 fest verbunden. Die dritte Crimpung C3 wird dabei in die Crimphülse 40 eingebracht, die somit den äußeren Geräteschlauch 62 gegen den geräteseitigen Abschnitt 13 der ersten Endhülse 10 klemmt.

Im Allgemeinen ermöglichen die Crimpverbindungen eine gasdichte Verbindung der Schläuche zum Schlauchverbinder. Dies gilt insbesondere für die erste Crimpung C1 und die zweite Crimpung C2, wobei die erste Crimpung C1 den äußeren Sondenschlauch 52 gasdicht mit dem Schlauchverbinder, insbesondere der ersten Endhülse 10, koppelt. Die zweite Crimpung C2 bewirkt eine gasdichte Kopplung zwischen der zweiten Endhülse 20, dem inneren Geräteschlauch 61 und (in den Figuren aus zeichnerischen Gründen nicht dargestellt) dem Düsenrohr 51 des Sondenschlauchs 50. Das Düsenrohr 51 bildet also eine Stützung für die zweite Crimpung C2, so dass der innere Gerätsschlauch 61 nicht abgeklemmt wird. Vielmehr wird der innere Geräteschlauch 61 gegenüber dem Düsenrohr 51 gasdicht verschlossen. Auf diese Weise wird eine gasdichte Gaszuführung über den inneren Geräteschlauch 61 und das Düsenrohr 51 bereitgestellt.

Die Gasrückführung erfolgt nach gasdichter Kontaktierung über den Schlauchverbinder, insbesondere die erste Crimpung C1 und die dritte Crimpung C3 (Fig. 3), über dem äußeren Sondenschlauch 52 und dem äußeren Geräteschlauch 62. Durch den Steg 30 zwischen den Endhülsen 10, 20 wird die Gasverbindung zwischen dem äußeren Geräteschlauch 62 und dem äußeren Sondenschlauch 52 ermöglicht, da der Steg 30 zwischen der ersten Endhülse 10 und der zweiten Endhülse 20 als Abstandshalter dient. Die erste weitere Crimpung C1' ermöglicht primär die elektrische Kontaktierung zwischen dem ersten Sondenleiter 53 und dem elektrisch leitenden Schlauchverbinder. Um eine gasdichte Verbindung zu gewährleisten, ist die erste Crimpung C1 vorgesehen, die in einem Bereich der ersten Endhülse 10 eingebracht wird, der frei von zusätzlichen Komponenten, insbesondere des ersten Sondenleiters 52, ist. Analog zu den Crimpverbindungen in der ersten Endhülse 10 kann auch in der zweiten Endhülse 20 eine doppelte Crimpung vorgesehen sein, wobei die zweite Crimpung C2 die gasdichte Verbindung zwischen dem inneren Geräteschlauch 61 und dem Düsenrohr 51 gewährleistet und eine weitere zweite Crimpung die Fixierung der elektrischen Kontakte, insbesondere des ersten und zweiten elektrischen Leiters 63, 64, sicherstellt.

Die elektrische Kontaktierung erfolgt vorzugsweise durch Klemmung der beiden parallelen elektrischen Leiter 63, 64 zwischen den inneren Geräteschlauch 61 des Geräteschlauchs 60 und den elektrisch leitfähigen Schlauchverbinder bzw. an das elektrisch leitfähige Düsenrohr 51. Andere elektrische Kontaktverbindungen, beispielsweise Lötverbindungen oder Wicklungen, sind möglich. Insbesondere der zweite elektrische Leiter 64 kann zur elektrischen Kontaktierung um das Düsenrohr 51 gewickelt werden.

Der erste elektrische Leiter 63 wird in dem in den Figuren dargestellten Ausführungsbeispiel durch die Bohrung 31 des Schlauchverbinders geführt und beim Einschieben des inneren Geräteschlauches 61 in die zweite Endhülse 20 mit der zweiten Endhülse 20 verklemmt und dadurch elektrisch kontaktiert. Durch die Crimpung des inneren Geräteschlauchs 61 auf das Düsenrohr 51 (Crimpung C2) im Bereich der zweiten Endhülse 20 wird die genannte Klemmung zwischen dem ersten elektrischen Leiter 63 und dem inneren Geräteschlauch 61 fixiert und gesichert. Gleichzeitig wird durch die Crimpung C2 eine Verbindung der koaxialen Gasleitungen, nämlich dem inneren Geräteschlauch 61 und dem Düsenrohr 51, realisiert.

Um den elektrischen Kontakt nach außerhalb des Gassystems herzustellen, wird der Strom über den Schlauchverbinder bzw. die Mehrfachcrimphülse auf den ersten Sondenleiter 53, der auf dem Außenumfang des äußeren Sondenschlauchs 52 angeordnet ist, durch Crimpung überführt (weitere erste Crimpung C1'). Auf diese Weise ist der elektrische Kontakt zwischen dem ersten elektrischen Leiter 63 und dem ersten Sondenleiter 53 hergestellt.

Der zweite elektrische Leiter 64 wird ebenfalls durch eine Klemmung fixiert und dadurch elektrisch mit dem Schlauchverbinder bzw. der Mehrfachcrimphülse kontaktiert. Durch die radiale Öffnung 65 im inneren Geräteschlauch 61 des Geräteschlauchs 60 wird der zweite elektrische Leiter 64 in das Lumen des inneren Geräteschlauchs 61 geführt. Beim darauffolgenden Einschieben des Düsenrohrs 51 in den inneren Geräteschlauch 61 wird der zweite elektrische Leiter 64 mit dem Düsenrohr 51 bzw. dem inneren Geräteschlauch 61 verklemmt und stellt somit den elektrischen Kontakt zum Düsenrohr 51 her, das den zweiten Sondenleiter 54 bildet. Über die Crimpung C2, die in der zweiten Endhülse 20 eingebracht wird, wird der elektrische Kontakt zwischen dem zweiten elektrischen Leiter 64 und dem Düsenrohr 51 bzw. dem zweiten Sondenleiter 54 fixiert. Das Düsenrohr 51 bildet also gleichzeitig den elektrisch leitenden zweiten Sondenleiter 54 und die innere Gasleitung, insbesondere Gaszuleitung, des Sondenschlauchs 50.

Mit dem beschriebenen Schlauchverbinder bzw. der Mehrfachcrimphülse wird ferner ermöglicht, die koaxiale Gasleitung des Geräteschlauchs 60 in eine koaxiale Gasleitung des Sondenschlauchs 50 zu überführen, wobei der Sondenschlauch 50 einen kleineren Querschnittsdurchmesser aufweist als der Geräteschlauch 60. Diese Durchmesseränderung ermöglicht den endoskopischen Einsatz des HF-Chirurgiegeräts. Dabei ist es zweckmäßig, wenn die Verbindungen zwischen dem Geräteschlauch 60 und dem Sondenschlauch 50 gasdicht und druckfest sind.

Der innere Geräteschlauch 61 wird dazu in der zweiten Endhülse 20 des Schlauchverbinders mit dem Düsenrohr 51 vercrimpt, wobei hier gleichzeitig zwei elektrische Kontaktierungen erfolgen (Crimpung C2). Der äußere Geräteschlauch 62 des Geräteschlauchs 60 wird über die Crimphülse 40 auf den Schlauchverbinder bzw. die Mehrfachcrimphülse gasdicht vercrimpt (Crimpung C3). Die Gasleitung von dem äußeren Geräteschlauch 60 zum äußeren Sondenschlauch 52 erfolgt über eine Öffnung in der Mehrfachcrimphülse bzw. im Schlauchverbinder, durch die auch das Düsenrohr 51 verläuft. Die Öffnung entspricht also dem Abstand zwischen den Endhülsen 10, 20, der durch den Steg 30 überbrückt wird. Die Öffnung bildet also eine Fluidverbindung zwischen dem Lumen des äußeren Gerätsschlauchs 62 und der ersten Endhülse 10. Durch die erste Endhülse 10 erfolgt sowohl die elektrische Kontaktierung auf den ersten Sondenleiter 53 mit der weiteren ersten Crimpung C1', als auch versetzt dazu die gasdichte Crimpung der Mehrfachcrimphülse bzw. des Schlauchverbinders auf den äußeren Sondenschlauch 52 (erste Crimpung C1).

Die Erfindung eignet sich insbesondere zur Verbindung von Schläuchen für HFchirurgische Geräte, insbesondere encloskopisch einsetzbare HF-Chirurgiegeräte. Ausführungsformen der Erfindung, die auf besondere Verbindungstechniken, beispielsweise Kleben oder Löten, verzichten, eignen sich insbesondere zur Verbindung von Schläuchen für HF-Chirurgiegeräte unter Reinraumbedingungen. Mit der Erfindung wird also eine reinraumtaugliche Schlauchverbindung bereitgestellt. Da der Schlauchverbinder vorteilhafterweise ein einziges Bauteil bildet, ist die Tauglichkeit als Einwegprodukt gewährleistet. Vorzugsweise umfasst der Schlauchverbinder bzw. die Mehrfachcrimphülse ein elektrisch leitendes Metall, so dass ferner sichergestellt wird, dass der Schlauchverbinder einem erhöhten Gasdruck standhält. Der Schlauchverbinder bildet also eine sichere, insbesondere gasdichte, Verbindung zwischen den Schläuchen eines HF-Chirurgiegeräts.

### Bezugszeichenliste

- 10: erste Endhülse
- 11: erster Durchgangskanal
- 12: sondenseitiger Abschnitt
- 13: geräteseitiger Abschnitt
- 14: Schulter
- 15: äußere Umfangsfläche
- 16: Axialbohrung
- 17: Anschlag
- 20: zweite Endhülse
- 21: zweiter Durchgangskanal
- 22: axiale Begrenzungsfläche
- 23: Endfläche
- 30: Steg
- 31: Bohrung
- 32: Absatz
- 33: Innenfläche
- 34: Außenfläche
- 40: Crimphülse
- 50: Sondenschlauch
- 51: Düsenrohr
- 52: äußerer Sondenschlauch
- 53: erster Sondenleiter
- 54: zweiter Sondenleiter
- 60: Geräteschlauch
- 61: innerer Geräteschlauch
- 62: äußerer Geräteschlauch
- 63: erster elektrischer Leiter
- 64: zweiter elektrischer Leiter
- 65: radiale Öffnung
- C1: erste Crimpung
- C1': weitere erste Crimpung
- C2: zweite Crimpung
- C3: dritte Crimpung

## Patentansprüche

1. Schlauchverbinder für ein Hochfrequenz-Chirurgiegerät mit zwei Endhülsen (10, 20), die jeweils einen axialen Durchgangskanal (11, 21) aufweisen und durch einen Steg (30) miteinander verbunden sind, der sich von der ersten Endhülse (10) axial in Richtung der zweiten Endhülse (20) erstreckt, wobei zumindest der Steg (30) wenigstens abschnittsweise ein elektrisch leitendes Material aufweist die Endhülsen (10, 20) zur Fixierung eines im jeweiligen Durchgangskanal (11, 21) anordnbaren Schlauches (50, 60) jeweils bereichsweise komprimierbar oder crimpbar sind.
und, **dadurch gekennzeichnet, dass**

2. Schlauchverbinder nach Anspruch 1,
**dadurch gekennzeichnet, dass**
die erste Endhülse (10) einen sondenseitigen Abschnitt (12) und einen geräteseitigen Abschnitt (13) umfasst, wobei der sondenseitige Abschnitt (12) einen kleineren Querschnittsdurchmesser als der geräteseitige Abschnitt (13) aufweist.

3. Schlauchverbinder nach Anspruch 1 oder 2,
**dadurch gekennzeichnet, dass**
die erste Endhülse (10), insbesondere der geräteseitige Abschnitt (13) einen größeren Querschnittsdurchmesser als die zweite Endhülse (20) aufweist.

4. Schlauchverbinder nach wenigstens einem der Ansprüche 1 bis 3,
**dadurch gekennzeichnet, dass**
die zweite Endhülse (20) und/oder der Steg (30) eine Bohrung (31) umfasst, die sich zumindest teilweise in radialer Richtung erstreckt.

5. Schlauchverbinder nach Anspruch 4,
**dadurch gekennzeichnet, dass**
die Bohrung (31) mit einer axialen Begrenzungsfläche (22) der zweiten Endhülse (20) fluchtet, die der ersten Endhülse (10) zugewandt ist.

6. Schlauchverbinder nach wenigstens einem der Ansprüche 2 bis 5,
**dadurch gekennzeichnet, dass**
die erste Endhülse (10) eine Schulter (14) umfasst, die zwischen dem sondenseitigen Abschnitt (12) und dem geräteseitigen Abschnitt (13) angeordnet ist
und einen größeren Querschnittsdurchmesser als der geräteseitige Abschnitt (13) aufweist.

7. Schlauchverbinder nach wenigstens einem der Ansprüche 1 bis 6,
**dadurch gekennzeichnet, dass**
eine äußere Crimphülse (40) vorgesehen ist, die den geräteseitigen Abschnitt (13) der ersten Endhülse (10) ringförmig umgreift und zur Fixierung eines geräteseitigen Schlauches (60) zumindest bereichsweise radial komprimierbar ist.

8. Schlauchverbinder nach wenigstens einem der Ansprüche 1 bis 7,
**dadurch gekennzeichnet, dass**
die zweite Endhülse (20) und/oder der Steg (30) wenigstens einen Lötbereich und/oder Klemmbereich umfasst.

9. Schlauchsystem für ein HF-Chirurgiegerät mit einem Schlauchverbinder gemäß Anspruch 1.

10. Verfahren zum Verbinden von Schläuchen für ein Hochfrequenz-Chirurgiegerät mit einem Schlauchverbinder gemäß Anspruch 1.

11. Verfahren gemäß Anspruch 10 umfassend die folgenden Schritte:
- ein Sondenschlauch (50) wird in einem ersten Durchgangskanal (11) der ersten Endhülse (10) und ein innerer Geräteschlauch (61) in einem zweiten Durchgangskanal (21) der zweiten Endhülse (20) angeordnet;
- ein erster elektrischer Leiter (63) wird zwischen der ersten Endhülse (10) und dem inneren Geräteschlauch (61) angeordnet, und
- in die erste Endhülse (10) werden wenigstens eine, insbesondere zwei, erste gasdichte Verbindungen und in die zweite Endhülse (20) wenigstens eine zweite gasdichte Verbindung eingebracht.

12. Verfahren nach Anspruch 11,
**dadurch gekennzeichnet, dass**
die erste gasdichte Verbindung eine erste Crimpung (C1) und/oder die zweite gasdichte Verbindung eine zweite Crimpung (C2) umfassen.

13. Verfahren nach Anspruch 10 oder 12,
**dadurch gekennzeichnet, dass**
der erste elektrische Leiter (63) über eine axiale Begrenzungsfläche (22) der zweiten Endhülse (20), die der ersten Endhülse (10) zugewandt ist, oder eine Bohrung (31), die sich zumindest teilweise in radialer Richtung durch die zweite Endhülse (20) oder den Steg (30) erstreckt, in den zweiten Durchgangskanal (21) eingeführt wird.

14. Verfahren nach wenigstens einem der Ansprüche 11 bis 13,
**dadurch gekennzeichnet, dass**
ein äußerer Geräteschlauch (62) über einen geräteseitigen Abschnitt (13) der ersten Endhülse (10) geführt und mit einer Crimphülse (40) umschlossen wird, in die zur Fixierung des äußeren Geräteschlauchs (62) eine dritte Crimpung (C3) eingebracht wird.

15. Verfahren nach wenigstens einem der Ansprüche 11 bis 14,
**dadurch gekennzeichnet, dass**
ein zweiter elektrischer Leiter (64) durch eine Öffnung (65) des inneren Geräteschlauchs (61) geführt und mit einem Düsenrohr (51) elektrisch verbunden wird, das sich koaxial von einem Sondenschlauch (50) in den inneren Geräteschlauch (61) erstreckt.

## Claims

1. Tube connector for an electrosurgical device with two end sleeves (10, 20) which each have an axial through-channel (11, 21) and are interconnected by a web (30) extending axially from the first end sleeve (10) in the direction of the second end sleeve (20), wherein at least the web (30) at least in sections has an electrically conductive material, **characterized in that** the end sleeves (10, 20) can each be compressed or crimped in sections in order to affix a tube (50, 60) which can be arranged in the respective through-channel (11, 21).

2. Tube connector according to Claim 1,
**characterized in that**
the first end sleeve (10) comprises a probe-side section (12) and a device-side section (13), with the probe-side section (12) having a smaller cross-sectional diameter than the device-side section (13).

3. Tube connector according to Claim 1 or 2,
**characterized in that**
the first end sleeve (10), more particularly the device-side section (13), has a larger cross-sectional diameter than the second end sleeve (20).

4. Tube connector according to at least one of Claims 1 to 3,
**characterized in that**
the second end sleeve (20) and/or the web (30) comprise a bore (31), which extends at least in part in the radial direction.

5. Tube connector according to claim 4,
**characterized in that**
the bore (31) is flush with an axial boundary face (22), facing the first end sleeve (10), of the second end sleeve (20).

6. Tube connector according to at least one of Claims 2 to 5,
**characterized in that**
the first end sleeve (10) comprises a shoulder (14), which is arranged between the probe-side section (12) and the device-side section (13) and has a larger cross-sectional diameter than the device-side section (13).

7. Tube connector according to at least one of Claims 1 to 6,
**characterized in that**
an outer crimping sleeve (40) is provided, which encompasses the device-side section (13) of the first end sleeve (10) in an annular manner and can, at least in regions, be compressed radially in order to affix a device-side tube (60).

8. Tube connector according to at least one of Claims 1 to 7,
**characterized in that**
the second end sleeve (20) and/or the web (30) comprise at least one soldering region and/or clamping region.

9. Tube system for an electrosurgical device with a tube connector according to Claim 1.

10. Method for connecting tubes for an electrosurgical device using a tube connector according to Claim 1.

11. Method according to Claim 10, comprising the following steps:
- a probe tube (50) is arranged in a first through-channel (11) of the first end sleeve (10) and an inner device tube (61) is arranged in a second through-channel (21) of the second end sleeve (20);
- a first electrical conductor (63) is arranged between the first end sleeve (10) and the inner device tube (61), and
- at least one, more particularly two, first gastight connections are introduced into the first end sleeve (10) and at least one second gastight connection is introduced into the second end sleeve (20).

12. Method according to Claim 11,
**characterized in that**
the first gastight connection comprises a first crimping (C1) and/or the second gastight connection comprises a second crimping (C2).

13. Method according to Claim 10 or 12,
**characterized in that**
the first electrical conductor (63) is inserted into the second through-channel (21) via an axial boundary face (22), facing the first end sleeve (10), of the second end sleeve (20) or a bore (31), which extends at least in part in the radial direction through the second end sleeve (20) or the web (30).

14. Method according to at least one of Claims 11 to 13,
**characterized in that**
an outer device tube (62) is routed via a device-side section (13) of the first end sleeve (10) and enclosed by a crimping sleeve (40), into which a third crimping (C3) is introduced in order to affix the outer device tube (62).

15. Device according to at least one of Claims 11 to 14,
**characterized in that**
a second electrical conductor (64) is routed through an opening (65) of the inner device tube (61) and electrically connected to a nozzle pipe (51), which extends coaxially from a probe tube (50) into the inner device tube (61).

## Revendications

1. Raccord pour tuyau flexible d'appareil chirurgical à haute fréquence, doté de deux douilles d'extrémité (10, 20) qui présentent chacune un canal axial de passage (11, 21) et qui sont reliées l'une à l'autre par une traverse (30) qui s'étend axialement dans la direction de la deuxième douille d'extrémité (20) en partant de la première douille d'extrémité (10), au moins la traverse (30) présentant au moins dans certaines parties un matériau électriquement conducteur,
**caractérisé en ce que**
certaines parties de chacune des douilles d'extrémité (10, 20) peuvent être comprimées ou serties pour fixer un tuyau flexible (50, 60) apte à être placé dans le canal de passage (11, 21) concerné.

2. Raccord pour tuyau flexible selon la revendication 1, **caractérisé en ce que** la première douille d'extrémité (10) comporte une partie (12) située côté sonde et une partie (13) située côté appareil, le diamètre de la section transversale de la partie (12) située côté sonde étant plus petit que celui de la partie (13) située côté appareil.

3. Raccord pour tuyau flexible selon les revendications 1 ou 2, **caractérisé en ce que** le diamètre de la section transversale de la première douille d'extrémité (10) et en particulier de la partie (13) située côté appareil est plus grand que celui de la deuxième douille d'extrémité (20).

4. Raccord pour tuyau flexible selon au moins l'une des revendications 1 à 3, **caractérisé en ce que** la deuxième douille d'extrémité (20) et/ou la traverse (30) comportent un alésage (31) qui s'étend au moins en partie dans la direction radiale.

5. Raccord pour tuyau flexible selon la revendication 4, **caractérisé en ce que** l'alésage (31) est aligné sur une surface frontière axiale (22), tournée vers la première douille d'extrémité (10), de la deuxième douille d'extrémité (20).

6. Raccord pour tuyau flexible selon au moins l'une des revendications 2 à 5, **caractérisé en ce que** la première douille d'extrémité (10) présente un épaulement (14) disposé entre la partie (12) située côté sonde et la partie (13) située côté appareil et **en ce que** le diamètre de sa section transversale est plus grand que celui de la partie (13) située côté appareil.

7. Raccord pour tuyau flexible selon au moins l'une des revendications 1 à 6, **caractérisé en ce qu'**une douille extérieure de sertissage (40) est prévue, laquelle entoure en anneau la partie (13) située côté appareil de la première douille d'extrémité (10) et peut être comprimée radialement au moins dans certaines parties pour fixer un tuyau flexible (60) situé côté appareil.

8. Raccord pour tuyau flexible selon au moins l'une des revendications 1 à 7, **caractérisé en ce que** la deuxième douille d'extrémité (20) et/ou la traverse (30) comportent au moins une partie brasée et/ou une partie serrée.

9. Système de tuyau flexible pour appareil chirurgical à HF, doté d'un raccord pour tuyau flexible selon la revendication 1.

10. Procédé pour relier des tuyaux flexibles d'un appareil chirurgical à haute fréquence à l'aide d'un raccord pour tuyau flexible selon la revendication 1.

11. Procédé selon la revendication 10, comportant les étapes suivantes :
- un tuyau flexible (50) de sonde est placé dans un premier canal de passage (11) de la première douille d'extrémité (10) et un tuyau flexible intérieur (61) d'appareil est placé dans un deuxième canal de passage (21) de la deuxième douille d'extrémité (20),
- un premier conducteur électrique (63) est placé entre la première douille d'extrémité (10) et le tuyau flexible intérieur (61) d'appareil et
- au moins une et en particulier deux premières liaisons étanches au gaz sont formées dans la première douille d'extrémité (10) et au moins une deuxième liaison étanche au gaz est formée dans la deuxième douille d'extrémité (20).

12. Procédé selon la revendication 11, **caractérisé en ce que** la première liaison étanche au gaz comprend un premier sertissage (C1) et/ou la deuxième liaison étanche au gaz comprend un deuxième sertissage (C2).

13. Procédé selon les revendications 10 ou 12, **caractérisé en ce que** le premier conducteur électrique (63) est inséré dans le deuxième canal de passage (21) par une surface frontière axiale (22) de la deuxième douille d'extrémité (20), tournée vers la première douille d'extrémité (10), ou par un alésage (31) qui s'étend au moins en partie dans la direction radiale dans la deuxième douille d'extrémité (20) ou dans la traverse (30).

14. Procédé selon au moins l'une des revendications 11 à 13, **caractérisé en ce qu'**un tuyau flexible extérieur (62) d'appareil est passé sur une partie (13) située côté appareil de la première douille d'extrémité (10) et est entouré par une douille de sertissage (40) dans laquelle un troisième sertissage (C3) est formé pour fixer le tuyau flexible extérieur (62) d'appareil.

15. Procédé selon au moins l'une des revendications 11 à 14, **caractérisé en ce qu'**un deuxième conducteur électrique (64) est passé par une ouverture (65) du tuyau flexible intérieur (61) d'appareil et est relié électriquement à un tube de tuyère (51) qui s'étend coaxialement depuis un tuyau flexible (50) de sonde jusque dans le tuyau flexible intérieur (61) d'appareil.
